# EUROPEAN PATENT SPECIFICATION

(11) **EP 0 417 865 B1**
(45) Date of publication and mention of the grant of the patent: **14.12.1994**
(21) Application number: 90202427.2
(22) Date of filing: 12.09.1990
(51) Int. Cl.: A61M 25/00, B29C 53/08

(54) **Drainage catheter and method for the manufacture thereof**
Drainagekatheter und Herstellungsverfahren dafür
Cathéter de drainage et son procédé de fabrication

(30) Priority: 13.09.1989 NL 8902286
(43) Date of publication of application: 20.03.1991
(73) Proprietor: CORDIS EUROPA N.V., NL-9301 LJ Roden (NL)
(72) Inventor: Venema, Hendrik Jan, NL-9301 PD Roden (NL); Wijkamp, Arnoldus Cornelis Johannes Maria, NL-9302 AW Roden (NL)
(74) Representative: 't Jong, Bastiaan Jacobus

(56) References cited:
- EP-A- 0 063 859
- US-A- 4 033 331
- US-A- 4 368 023
- US-A- 4 735 620

## Description

The invention relates to a method for the manufacture of a drainage catheter such as a nephrostomy catheter comprising the extrusion from flexible plastic of a tubular basic body with a central channel, the attachment to one end of the basic body of a coupling element, the deformation of the opposite end part into a curved form and the arrangement in the curved end part of a number of side-openings connecting the central channel to the outside.

A drainage catheter, in particular a nephrostomy catheter, manufactured according to this per se known method is brought into an elastic, deformed and straightened state for insertion into the patient. The known drainage catheters have the drawback that they can have only small discharge openings because otherwise the wall in the vicinity of the side-openings may form sharp outer ridges. When the side-openings are arranged in the end part, for example by punching, while the latter is in the curved form, sharp ridges occur when straightening takes place, and when the openings are punched in the straightened form the wall buckles outward sharply in the curved state of the catheter end.

Since the sharply buckled wall portion causes danger of injury either when it is being inserted into the patient or in the inserted position it is necessary to avoid this buckling and small side-openings are therefore used. The drainage capacity of the catheter is however limited as a result and small drainage openings are moreover more quickly obstructed by blockage.

The invention now has for its object to provide a method for the manufacture of a drainage catheter as described above whereby large side-openings can be employed without the danger occurring of buckling of the wall in the vicinity of these side-openings.

With the method according to the invention this object is achieved in that the basic body is extruded such that the central channel comes to lie eccentrically in the basic body, that during the plastic deformation of the end part the basic body is supported for free rotation about its lengthwise axis and that the arrangement of the side-openings takes place after the end part has been plastically deformed into the curved state. The buckling of the wall portions does not occur with the catheter manufactured in this manner because during straightening the thin wall portion defined by the eccentric location of the central channel is straightened while the thick wall portion does not crumple inward and buckle sharply outward because of its greater stiffness. The condition for achieving this is that the thin wall portion is situated on the inside of the bend while the thick wall portion is situated on the outside. In a surprising yet in retrospect explicable manner it has been found that by supporting the basic body for free rotation about its lengthwise axis during the plastic deformation of the end part, the basic body automatically rotates such that the thick wall portion comes to lie on the outside of the bend and the thin wall portion on the inside of the bend. The method according to the invention is simple to perform and provides a drainage catheter which can be provided with large side-openings without there being the slightest risk that the wall portions in the vicinity of the side-openings will form sharp outward ridges.

According to a preferred embodiment of the method according to the invention the step of claim 2 is applied. As a result the central channel debouches centrally into the pointed end of the catheter.

The method according to the invention can be performed in a simple manner by application of the steps of claim 3. Use of a metal thread with the desired curved form achieves in a simple manner that the basic body is supported for free rotation about its lengthwise axis during deformation.

The invention also relates to and provides a drainage catheter such as a nephrostomy catheter comprising a flexible, tubular basic body with a central channel which is fixed with one end in a coupling element and comprises at the opposite end a plastically deformed curved part, whereby in the curved part is arranged a number of side-openings connecting the central channel to the outside. According to the invention, at least in the curved end part wherein the side-openings are arranged the central channel lies shifted eccentrically toward the inner side of the bend. Thus achieved in the above described manner is that when the catheter is straightened the wall portions in the vicinity of the side openings do not buckle sharply outward.

From US-A-4 033 331 a catheter having an excentric central channel and side wall openings arranged therein is known as such. The central openings are arranged adjacent the distal end in a not or only slightly curved end portion. The catheter has a second excentric channel for a contour or stiffening wire.

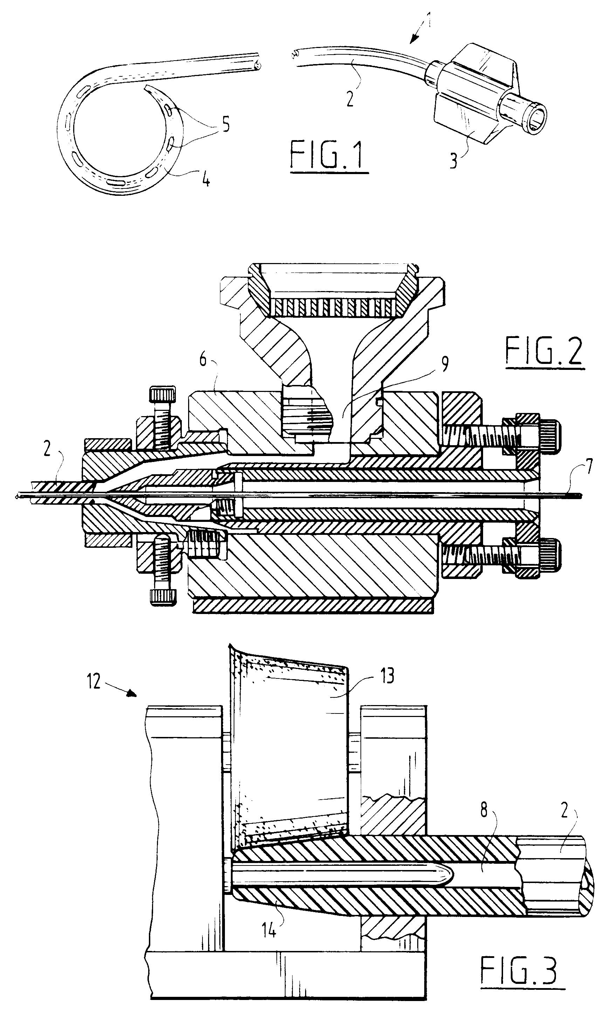

The invention will be further elucidated in the following description with reference to the annexed figures. Fig. 1 shows in partly perspective view a catheter manufactured by the method according to the invention;
fig. 2 shows a schematic section of an extrusion head as used with the method according to the invention;
fig. 3 is a partly broken away perspective view for the elucidation of the method step for forming a point on the catheter;
fig. 4 is a perspective view for the elucidation of the plastic deformation of the end part of the catheter;
fig. 5 is a partly broken away perspective view for the elucidation of the method step for arranging the side-openings;
fig. 6 shows a cross-sectional view of the catheter along the line VI-VI in fig. 5.

The present invention relates to a catheter of the type shown in fig. 1. It relates to a drainage catheter such as a nephrostomy catheter. This catheter 1 comprises a basic body 2 of flexible plastic. The basic body 2 has a central channel. Attached to one end of the basic body 2 is a coupling element 3. The opposite end part 4 is plastically deformed into a curved shape. In this plastically deformed, curved end part a number of side-openings 5 are formed. When the catheter is introduced into a patient the curved end part 4 is elastically straightened and when the catheter has been placed in position tensioning of the end part 4 is released and it again assumes its curved form.

As fig. 2 shows the tubular basic body 2 is extruded in a per se known manner. Fig. 2 shows schematically an extrusion head 6 into which flexible plastic in plastic form is fed under pressure at 9. This plastic leaves the extrusion head 6 on the left-hand side in coherent form, this being in the form of the flexible, tubular material for the basic body 2.

A core thread around which the plastic is sprayed is introduced eccentrically into the extrusion head. This core thread is later removed.

The material for the basic body 2 can thus be extruded in greater lengths whereby for each catheter to be manufactured a piece of the material can be cut off at the appropriate length.

As fig. 3 shows, after cutting off the basic body 2 from the starting material, one end thereof is given a point. This takes place using a grinding device 12 comprising a grindstone 13. During grinding of the point 14 the basic body 2 is centred on the central channel 8. This is carried out by providing the grinding device 12 with a pin onto which the basic body is pushed with the central body 8. Centring of the basic body 2 on the central channel 8 during the point forming achieves that the debouchment of the central channel at the outermost point comes to lie concentrically to the pointing 14.

After forming of the point, the end part 4 of the basic body 2 is plastically deformed into the curved form shown. To this end a metal thread 16 with the required curved from is pushed into the central channel 8. The end part 4 thus deforms elastically into the required form. To make this form permanent the end part 4 is heated with the metal thread 16 therein to close to the softening temperature of the material of the basic body 2. The end part 4 having the metal thread 16 still therein is then cooled so that the curved form is maintained in the end part 4. The metal shaping thread 16 can now be removed and the end part 4 springs back into the curved form.

Because during the plastic deformation of the end part 4 the basic body is supported for free rotation about its lengthwise axis by the metal thread 16 the end part automatically assumes the position which corresponds with the state of minimum energy. This means that the thickest portion of the diameter of the end part 4 comes to lie on the greatest possible radius and therefore that the thick wall portion 17 comes to lie on the outer side of the bend and the thin wall portion 18 comes to lie on the inside of the bend. After the end part 4 has been given a curved form in this way the side-openings can be punched therein.

As fig. 5 shows, for this purpose the end part 4 is laid in a mould 21 in which is arranged a fixing groove 22 corresponding with the curved shape of the end part 4.

A punch 24 is attached to an up and downward movable holder and lies eccentrically relative to the mould 21, which is rotatable around the shaft 23. Inserted into the central channel of the catheter is a nylon thread which serves as stop for the punch 24. One hole 5 is punched at a time. The mould is then turned to the following position and the next hole can be punched. When one side is finished the mould with the catheter is turned over and the other side is punched. After punching the nylon thread is removed.

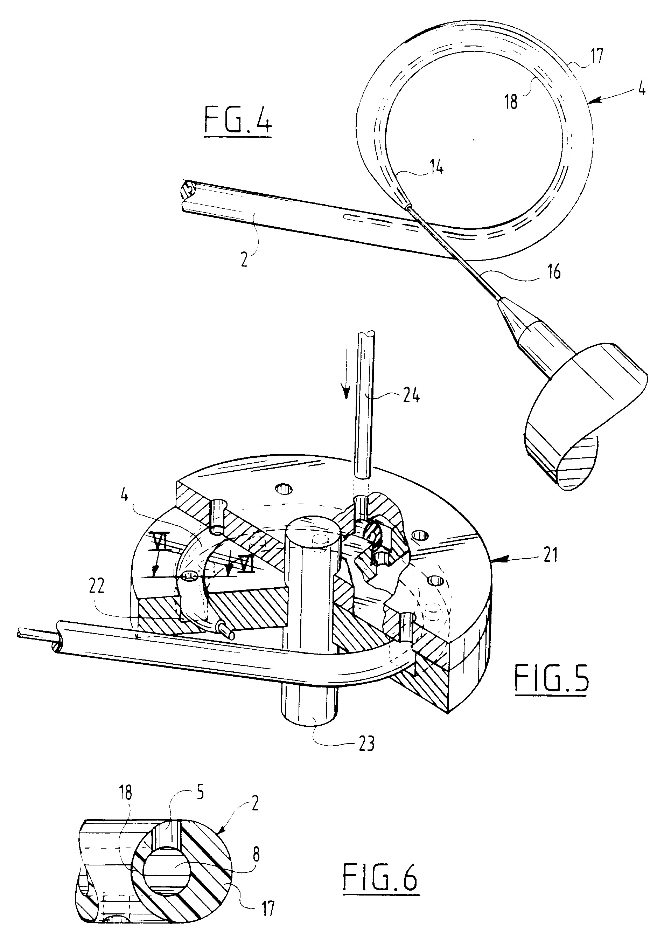

As fig. 6 shows, the punch blade 24 is positioned such that it lies directly above the central channel in the end part 4.

Because the thick wall portion 17 lies on the outer side in the curved end part, when this end part is straightened the thin wall portion 18 which is located on the inner side of the bend is subject to tensile loading and the thick wall portion 17 which is located on the outer side of the bend is subject to pressure loading. The thin wall portion does not of course buckle sharply outward when straightened because of the tensile loading. The thick wall portion does not however buckle either because of the greater stiffness. The side-openings 5 can therefore take a relatively large form.

## Claims

1. Method for the manufacture of a drainage catheter (1) such as a nephrostomy catheter comprising the extrusion from flexible plastic of a tubular basic body (2) with a central channel (8), the attachment to one end of said basic body of a coupling element (3), the deformation of the opposite end part (4) into a curved form and the arrangement in the curved end part of a number of side-openings (5) connecting said central channel (8) to the outside, **characterized in that** said basic body (2) is extruded such that said central channel comes to lie eccentrically in said basic body (2), that during the plastic deformation of said end part (4) said basic body (2) is supported for free rotation along its lengthwise axis and that the arranging of the side openings is carried out after said end part has been plastically deformed into the curved shape.

2. Method as claimed in claim 1, **characterized in that** the point (14) of the curved end part is pointed by conical grinding whereby said end part (4) is centred on the central channel (8).

3. Method as claimed in claim 1, **characterized in that** the plastic deformation of the end part (4) is performed by pushing a metal thread (16) with the required curved form into the central channel (8), by heating said end part with (4) said metal thread (16) therein to close to softening temperature, and by subsequent cooling and removing of said metal thread (16).

4. Drainage catheter (1) such as a nephrostomy catheter comprising a flexible, tubular basic body (2) with a central channel (8) which is attached at one end in a coupling element (3) and which comprises at the opposite end a plastically deformed curved part (4) whereby in said curved part (4) a number of side-openings (5) are arranged connecting said central channel (8) to the outside, **characterized in that** at least in the curved end part (4) wherein side-openings (5) are arranged a single central channel (8) lies shifted eccentrically toward the inside of the bend.

## Patentansprüche

1. Verfahren zur Herstellung eines Drainagekatheters (1) beispielsweise eines Nephrostomie-Katheters, das umfaßt: das Extrudieren eines röhrenförmigen Grundkörpers (2) aus flexiblem Kunststoff mit einem Mittelkanal (8), das Befestigen eines Kupplungselements (3) an einem Ende dieses Grundkörpers, das Umformen des gegenüberliegenden Endteils (4) in eine gebogene Form und das Anordnen einer Anzahl von seitlichen Öffnungen (5) im gebogenen Endteil, die den Mittelkanal (8) nach außen verbinden, dadurch gekennzeichnet, daß der Grundkörper (2) so extrudiert wird, daß der Mittelkanal in ihm exzentrisch liegt, daß während der plastischen Umformung des Endteils (4) dieser Grundkörper (2) um seine Längsachse frei drehbar gelagert wird, und daß das Anordnen der seitlichen Öffnungen ausgeführt wird, nachdem der Endteil in die gebogene Form plastisch umgeformt worden ist.

2. Verfahren nach Anspruch 1, dadurch gekennzeichnet, daß die Spitze (14) des gebogenen Endteils durch konisches Schleifen zugespitzt wird, wodurch der Endteil (4) auf den Mittelkanal (8) zentriert wird.

3. Verfahren nach Anspruch 1, dadurch gekennzeichnet, daß die plastische Umformung des Endteils (4) ausgeführt wird durch Schieben eines Metallfadens (16) mit der erforderlichen gebogenen Form in den Mittelkanal (8), durch Erwärmen des Endteils (4) mit diesem Metallfaden (16) darin bis nahe der Erweichungstemperatur, und durch nachfolgendes Kühlen und Entfernen des Metallfadens (16).

4. Drainagekatheter (1), beispielsweise Nephrostomie-Katheter, der einen flexiblen, rohrförmigen Grundkörper (2) mit einem Mittelkanal (8) aufweist, der an einem Ende in einem Kupplungselement (3) befestigt ist und am gegenüberliegenden Ende ein plastisch umgeformtes, gebogenes Teil (4) aufweist, wobei in diesem gebogenen Teil (4) eine Anzahl von seitlichen Öffnungen (5) angeordnet ist, die den Mittelkanal (8) nach außen verbinden, dadurch gekennzeichnet, daß mindestens im gebogenen Endteil (4), in dem seitliche Öffnungen (5) angeordnet sind, ein Mittelkanal (8) verläuft, der exzentrisch in Richtung der Innenseite der Biegung verschoben ist.

## Revendications

1. Procédé de fabrication d'un cathéter de drainage (1), tel qu'un cathéter de néphrostomie, comprenant l'extrusion, à partir d'une matière plastique souple, d'un corps de base (2) tubulaire avec un canal central (8), la fixation d'un élément de couplage (3) à une extrémité dudit corps de base, la déformation de la partie extrémité (4) opposée afin d'obtenir une forme incurvée et la formation dans la partie extrémité incurvée d'un certain nombre d'ouvertures latérales (5), reliant ledit canal central (8) à l'extérieur, caractérisé en ce que ledit corps de base (2) est extrudé de manière à ce que ledit canal central soit situé de façon excentrique dans ledit corps de base (2), en ce que, durant la déformation plastique de ladite partie extrémité (4), ledit corps de base (2) est supporté de façon à tourner librement le long de son axe longitudinal et en ce que l'agencement des ouvertures latérales est effectué après que ladite partie extrémité ait été déformée plastiquement afin d'obtenir la forme incurvée.

2. Procédé selon la revendication 1, caractérisé en ce que la pointe (14) de la partie d'extrémité incurvée est rendue pointue par un meulage conique, de manière à ce que ladite partie extrémité (4) soit centrée sur le canal central (8).

3. Procédé selon la revendication 1, caractérisé en ce que la déformation plastique de la partie extrémité (4) est effectuée en poussant un fil métallique (16) ayant la forme incurvée souhaitée dans le canal central (8), en chauffant ladite partie extrémité (4) contenant en son sein ledit fil métallique (16), à une température proche de la température de ramollissement et en effectuant un refroidissement successif et en retirant ledit fil métallique (16).

4. Cathéter de drainage (1) tel qu'un cathéter de néphrostomie, comprenant un corps de base (2) tubulaire flexible ayant un canal central (8) qui est fixé par une extrémité dans un élément de couplage (3) et comprenant, à l'extrémité opposée, une partie incurvée (4) déformée plastiquement, de manière que dans ladite partie incurvée (4) un certain nombre d'ouvertures latérales (5) soient formées, reliant ledit canal central (8) à l'extérieur, caractérisé en ce qu'au moins la partie extrémité (4) incurvée, dans laquelle sont ménagées les ouvertures latérales (5), un canal central (8) unique se situe de façon décalée excentriquement vers l'intérieur de l'incurvation.
